# EUROPEAN PATENT SPECIFICATION

(11) **EP 4 651 831 B1**
(45) Date of publication and mention of the grant of the patent: **09.12.2026**
(21) Application number: 24708260.5
(22) Date of filing: 09.01.2024
(51) Int. Cl.: A61F 2/18, A61L 27/04, A61L 27/06, A61L 27/36

(54) **IMPROVED STEM OF THE PROSTHESIS FOR THE RECONSTRUCTION OF THE OSSICULAR CHAIN**
VERBESSERTER SCHAFT DER PROTHESE ZUR REKONSTRUKTION DER GEHÖRKNÖCHELCHENKETTE
TIGE AMÉLIORÉE DE LA PROTHÈSE POUR LA RECONSTRUCTION DE LA CHAÎNE OSSICULAIRE

(30) Priority: 17.01.2023 IT 202300000570
(43) Date of publication of application: 26.11.2025
(73) Proprietor: Malafronte, Giuseppe, 80122 Napoli (IT)
(72) Inventor: Malafronte, Giuseppe, 80122 Napoli (IT)
(74) Representative: Fezzardi, Antonio
(86) International application number: PCT/IT2024/050002
(87) International publication number: WO 2024/154161

(56) References cited:
- US-A- 4 641 651
- "Prostheses: Design, Types and Complications", 31 December 2012, NOVA SCIENCE PUBLISHERS, Hauppauge, ISBN: 978-1-62100-854-5, article MALAFRONTE GIUSEPPE: "Prostheses for middle ear ossicular chain recontruction", pages: 83 - 95, XP093061888

## Description

The present invention concerns the surgical sector, with particular reference to ear pathologies.

The tympanic cavity is an air cavity that communicates with the nasal cavities thanks to the Eustachian tube. The tympanic cavity contains three ossicles: hammer, incus and stapes, which make up the so-called "ossicular chain". The aim of the ossicular chain is to transport sound from the tympanic membrane to the cochlea. In the latter, the mechanical energy of sound is transformed into electrical energy, that is, into electrical pulses which, through the auditory nerve, are sent to the brain. A damage to the ossicular chain causes a reduction in hearing ability, called transmission hearing loss.

The operation to reconstruct the ossicular chain is called "ossiculoplasty". Based on the broken ossicles we have two types of ossiculoplasty:
- *partial* ossiculoplasty, when only one ossicle is damaged, i.e. the incus;
- *total* ossiculoplasty, when two small ossicles are damaged, namely the incus and the superstructure of the stapes.

The prosthesis used in partial ossiculoplasty is called the partial ossicular replacement prosthesis (PORP). The PORP is placed between the tympanic membrane and the stapes capital and replaces the damaged incus. The prosthesis used in total ossiculoplasty is called the total ossicular replacement prosthesis (TORP). The TORP is placed between the tympanic membrane and the plate of the stapes and replaces the damaged incus and the superstructure of the stapes.

It is known from US 4641651, a TORP comprising a head which is formed from a cartilaginous block and a synthetic stem of cylindrical shape which is provided with a tip at one end.

A better understanding of the invention will be provided by the following detailed description and with reference to the attached figures which illustrate some already known technical solutions, as well as a preferred embodiment of the invention, illustrated and described here by way of example and not by way of limitation.

In the drawings:

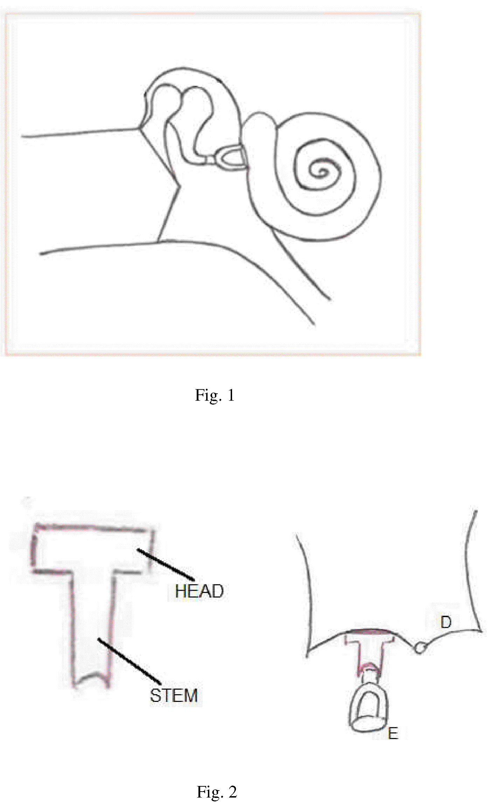

figure 1 schematically shows the ossicular chain of a human ear;
figure 2 shows respectively: on the left the shape of a synthetic PORP of a known type, and on the right its positioning between the tympanum and the stapes;
figure 3 shows respectively on the left the shape of a synthetic TORP of a known type and on the right its positioning between the tympanum and the plate of the stapes;
figure 4 shows a prosthesis for stapes surgery (1) and the same prosthesis modified (2) to create a semi-synthetic static TORP of a known type;
figure 5 illustrates how a 5 mm long semi-synthetic TORP of known type is made and its positioning for total ossiculoplasty;
figures 6 and 7, similar to fig. 5, are related to a semi-synthetic TORP of 6 mm and 7 mm length, respectively;
figure 8 illustrates how an improved TORP according to the present invention is constructed having an overall length of 5 mm;
figure 9 illustrates how to construct an improved TORP according to the present invention having an overall length of 6 mm;
figure 10 illustrates how to construct an improved TORP according to the present invention having an overall length of 7 mm;
figures 11A and 11B illustrate the invention placed between the tympanum and the plate for the total reconstruction of the ossicular chain (incus and superstructure of the damaged staples), as well as its behavior in the event of variations in atmospheric pressure;
figure 12 illustrates the invention placed between the tympanum and the plate of an intact stapes for the reconstruction of the ossicular chain, when only one ossicle (incus) is damaged.

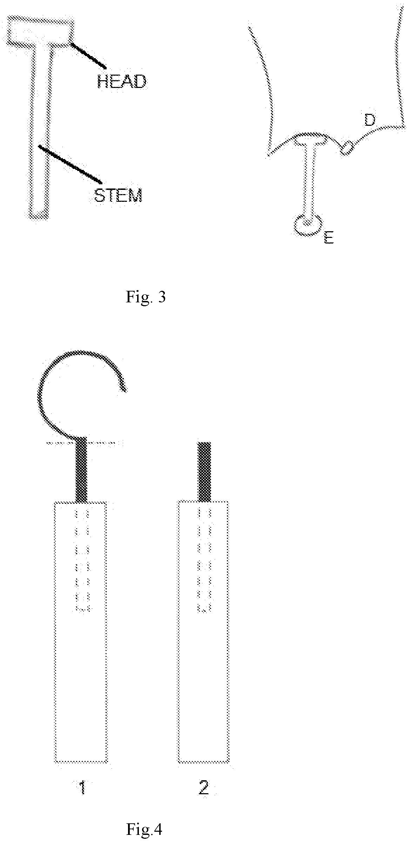

TORPs currently in use are synthetic in nature. They can be made of gold, platinum, hydroxyapatite or titanium. Each of these TORPs consists of a flat head with a diameter of approximately 2,5 mm placed at the end of a cylindrical stem (fig. 3). usually the distance between the tympanic membrane and the plate varies from 5 to 7 mm, in rare cases it is 8 mm. TORPs most used in the reconstruction of the ossicular chain have a total length of 5 or 6 or 7 mm.

The drawbacks of the current TORPs are essentially four:
1-They do not have a good anchoring to the tympanic membrane;
2-They do not have a stable anchoring to the plate of the stapes;
3-They are unable to change their length based on the movements of the tympanic membrane;
4-They do not have a high biocompatibility in hostile environments.

The reduction in atmospheric pressure (air travels, mountain excursions) determines an increase in the pressure present in the tympanic cavity, with a displacement of the tympanic membrane of 1 or 1,5 millimeters towards the outside. The prostheses on the market are static prostheses, as they cannot change their length, they easily lose contact with the tympanic membrane and the plate and become dislocated. similarly, an increase in atmospheric pressure (underwater activity) or tubal dysfunctions determine a reduction in the pressure present in the tympanic cavity, with an inward movement of the tympanic membrane. The increase in tension between the tympanum and the synthetic prosthesis causes the extrusion of the prosthesis itself. The extrusion, or dislocation, of this prosthesis determines the reappearance of the transmission hear loss.

These are the main reasons why, in the long term, the extrusion or dislocation rate of synthetic TORP is high (35-55%). To solve or at least reduce these reappearance rates of transmission hear loss, in 2007 the same Applicant proposed and published a new type of TORP called "semi-synthetic TORP" (hereinafter also called ToRPss).

This TORPss is made by assembling a cylindrical titanium and polytetrafluoroethylene stem with an autologous cartilaginous head. To create the stem of this TORPss, the base of the hook of a prosthesis is cut - in fact of a known type (made of titanium and polytetrafluoroethylene) - for stapes surgery (fig. 4A) with a total length of 7 mm.

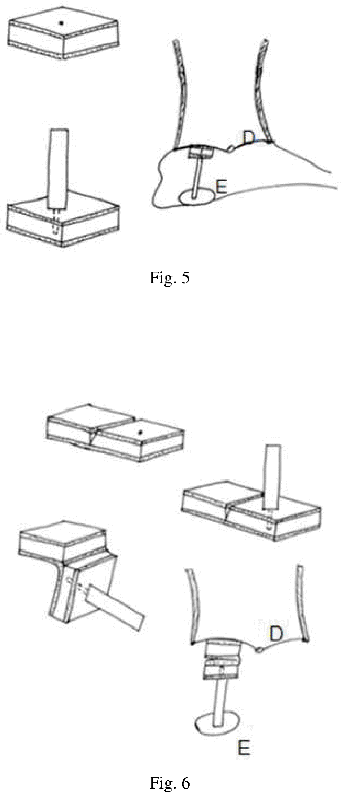

This results in a cylindrical stem made of titanium and polytetrafluoroethylene with a diameter of 0,4 mm and a length of 4 mm. This stem is provided with a blunt titanium tip with a diameter of 0,2 mm and 1 mm in length (fig. 4B). The blunt tip is completely inserted into the cartilaginous head (fig. 5). In particular, the head of the TORPss can be made of one, two or three blocks of tragus cartilage held together by a perichondrium hinge (figs. 5, 6, 7). The tragus cartilage is ideal for building blocks as it is flat and has a thickness of 1 mm. The 5 mm long TORPss has a head made of a single 2.5x2.5 mm square block of cartilage covered on both sides by perichondrium. In the center of the block, only in the perichondrium, a hole is made with an insulin needle. The blunt tip of the stem is then inserted into the hole in the perichondrium: by exerting a small pressure the tip penetrates completely into the cartilage. This results in a 5 mm long TORPss (fig. 5). The assembly of the head cartilaginous block with the stem appears to be very stable. The total reconstruction of the ossicular chain is obtained by placing the cartilaginous head under the tympanic membrane and the base of the synthetic stem above the plate (fig. 5).

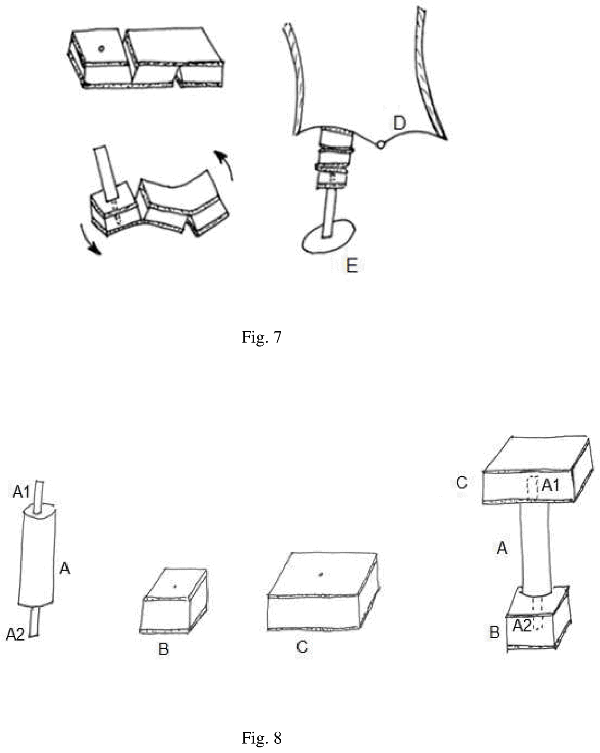

The 6 mm long TORPss is obtained by assembling the stem with two cartilaginous blocks, held together by a perichondrium hinge (fig. 6). similarly, the 7 mm long TORPss is obtained by assembling the stem with three cartilaginous blocks, held together by a perichondrium (fig. 7).

However, it is necessary to note that the TORPss has only solved two of the four drawbacks of the synthetic TORP, as in fact:
1-The head of the TORPss has a good anchoring to the tympanum as the perichondrium of the cartilaginous block in contact with the tympanic membrane, during the healing process, is welded to the tympanic membrane and does not detach from the tympanum even in the presence of pathologies;
2- The base of the TORPss does not have a good anchoring to the plate of the stapes;
3- The TORPss is a static prosthesis, not being capable to modify its length based on the movements of the tympanic membrane;
4- The TORPss has a high biocompatibility in a hostile environment as the cartilaginous head avoids contact of the synthetic stem with the tympanic membrane and prevents its extrusion in the presence of pathologies.

The percentage of extrusion of the TORPss and its dislocation from the tympanic membrane is very low, however the problem of the dislocation of the base of the stem from the plate of the stapes remains.

In essence, drawbacks of the TORPss are:
1-the construction of the cartilaginous head of the TORPss, which is simple in the 5 mm long TORPss as it is formed by a single cartilaginous block, is more difficult in the 6 or 7 mm long TORPss as it is formed by two or three cartilaginous blocks held together by a cartilaginous hinge: its construction requires a lot of experience from the operator.
2- it is a static prosthesis, not being capable to increase its length when the distance between the membrane and the plate increases during variations in atmospheric pressure.
3- the lack of anchoring of the base of the synthetic stem to the plate of the stapes, and the inability to increase its length have the disadvantage that the stem, not anchored, can easily dislocate from the plate - especially during variations in atmospheric pressure - causing the reappearance of a transmission hearing loss.

Main purpose of the present invention is to overcome these drawbacks, providing an innovative improved TORP compared to the traditional TORPss described above.

The improved TORP according to the invention is characterized in that it provides, in combination:
- a head which is formed by a cartilaginous block covered by perichondrium (1 mm thick and a surface area of 2.5x 2.5 mm) the construction of which is very simple (figs. 8, 9, 10);
- a new synthetic stem with a cylindrical shape which is provided, on both sides, with a respective blunt titanium tip 1 mm long and 0.2 mm in diameter (figs. 8, 9, 10);
- a base which is formed by a cartilaginous block covered by a perichondrium (1 mm thick and by a surface area of 0.8x 0.8 mm (figs. 8, 9, 10);
in which the different length of the semi-synthetic prosthesis is obtained by providing cylindrical synthetic stems of different lengths.

According to the present invention, the tip (A1) of one end of the stem (A) is entirely inserted into the head (C) of the prosthesis which is formed by a cartilaginous block covered by a perichondrium, while the tip (A2) of the opposite end of the stem (A) is entirely inserted into the base (B) of the prosthesis, which is formed by a cartilaginous block smaller than that of the head (figs. 8, 9, 10).

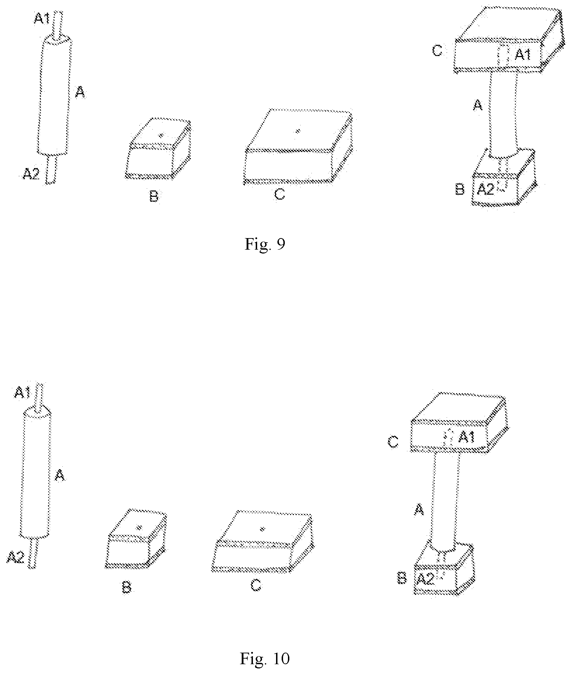

Therefore, with reference to figures 8, 9 and 10, the new improved TORP consists of:
- a cartilaginous head 1 mm thick and with a surface area of 2.5x 2.5 mm;
- a synthetic cylindrical stem (in gold, platinum, titanium and polytetrafluoroethylene), with a diameter of 0.4 mm and having on each of the two ends a blunt tip 1 mm long and with a diameter of 0.2 mm, said stem having a length equal to:
   ∘ 3 mm for the 5 mm long TORP (fig. 8);
   ∘ 4 mm for the 6 mm long TORP (fig. 9);
   ∘ 5 mm for the 7 mm long TORP (fig. 10);
- a cartilaginous base 1 mm thick and with a surface area of 0.8x0.8 mm.

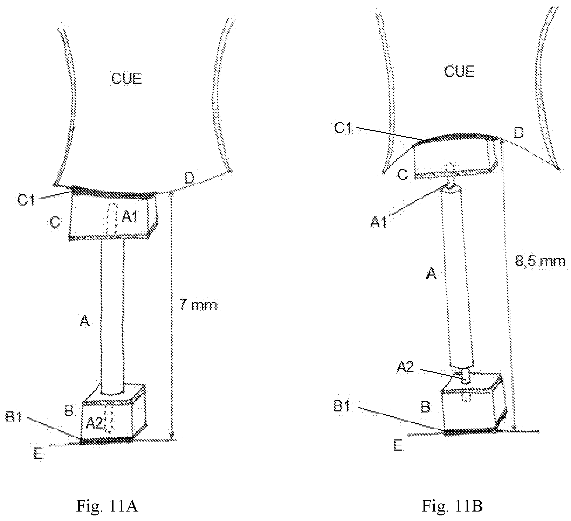

In the reconstruction of the ossicular chain, the head of the new prosthesis according to the present invention is placed under the tympanic membrane and its base above the plate of the stapes (fig. 11A).

Advantageously, during the healing process, the perichondrium of the head is welded to the tympanic membrane and the perichondrium of the base is welded to the mucosa of the plate. For this reason, the improved TORP that is described is very stable as it has two solid anchors: one with the tympanum and the other with the plate. On the contrary, the traditional TORPss is less stable as it has only one anchor: the one with the tympanum.

Furthermore, the TORP according to the invention is simpler to construct than the traditional TORPss, and allows for a more stable reconstruction of the ossicular chain, even in the event of variations in atmospheric pressure which cause an increase in the distance between the tympanic membrane and the plate. In these events, in fact, the blunt tips at the two ends still remain partially inserted in the respective holes of the head and the base, without causing dislocations (fig. 11A and 11B).

Finally, Fig. 11a shows the semi-synthetic dynamic prosthesis which is the object of the present invention, placed between the tympanic membrane (D) and the plate (E).

Fig. 11b shows the behavior of the aforementioned prosthesis in the event of a displacement of the tympanic membrane (D) in the external auditory canal (CUE) with an increase in the distance between the tympanic membrane (D) and the plate (E), with a partial protrusion of the tip (A1) of the stem (A) of the prosthesis head (C) and a partial protrusion of the tip (A2) of the stem (A) from the base of the prosthesis (B).

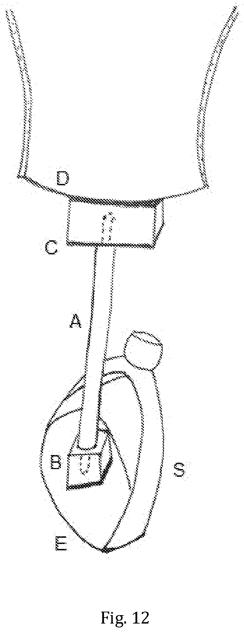

Fig. 12 shows the interposition of the semi-synthetic dynamic prosthesis between the tympanic membrane (D) and the plate (E) of the intact stapes (S).

### LEGEND:

A: synthetic stem 3 or 4 or 5 millimeters long
   A1: tip of the stem (1 mm long) to be inserted into the head of the prosthesis
B: base of the prosthesis formed by a cartilaginous block with perichondrium (1 mm thick; 0.8x 0.8 mm in surface)
C: head of the prosthesis formed by a cartilaginous block with perichondrium (1 mm thick; 2.5x 2.5 mm in surface)

A: synthetic stem of the prosthesis
   A1: tip of the stem completely inserted into the head
   A2: tip of the stem completely inserted into the base
B: base of the prosthesis made of cartilage covered by perichondrium
   B1: perichondrium of the base welded to the plate E
C: head of the prosthesis made of cartilage covered by perichondrium
   C1: perichondrium of the head welded to the tympanic membrane D
D: tympanic membrane
E: plate
CUE: external auditory canal

## Claims

1. A total ossicular replacement prosthesis (TORP) for the reconstruction of the ossicular chain, comprising a head and a synthetic stem, wherein
- said head (C) is formed by a single cartilaginous block and is provided with a hole;
- said improved TORP further comprises a cartilaginous base (B), provided with a hole, having the same thickness as the head (C) but with a smaller surface;
- said synthetic stem (A) has a cylindrical shape and is provided, on both sides, with a respective blunt tip (A1, A2), in which said blunt tips (A1, A2) are inserted in the respective holes of the head (C) and of the base (B);
said TORP is configured in such a way as to be able to position, respectively, said head (C) under the tympanic membrane (D) and said base (B) above the plate (E) of the stapes (s); thus obtaining that during the healing process the perichondrium of the head (C) is bonded to the tympanic membrane (D) and the perichondrium of the base (B) is bonded to the mucosa of the plate (E).

2. The TORP according to the previous claim, wherein the tip (A1) of one end of the stem (A) is configured to be completely inserted into said head (C) which is formed by a cartilaginous block covered by a perichondrium, while the tip (A2) of the opposite end of the stem (A) is configured to be completely inserted into said base (B), which is formed by a cartilagineous block smaller than that of the head (C).

3. The TORP according to one of the preceding claims, wherein it is 5 or 6 or 7 mm long and consists of:
• a cartilaginous head (C) covered by a perichondrium with a thickness of 1 mm and with a surface area of 2,5 x 2,5 mm;
• a synthetic cylindrical stem (A) either in gold, or platinum, or titanium, or titanium and polytetrafluoroethylene, with a diameter of 0.4 mm and having a 1 mm long blunt tip (A1, A2) on each of the two ends (A1, A2) and with a diameter of 0.2 mm, said stem (A) having a length equal to:
- 3 mm for the 5 mm long TORP,
- 4 mm for the 6 mm long TORP,
- 5 mm for the 7 mm long TORP;
• a cartilaginous base (B) covered by a perichondrium with a thickness of 1 mm and a surface area of 0.8x 0.8 mm.

4. The TORP according to one of the preceding claims, wherein it is configured in such a way that the length of the blunt tips (A1, A2) is such that, in the event of variations in atmospheric pressure, said blunt titanium tips (A1, A2) present at the two ends remain in any case at least partially inserted in the respective holes of the head and the base, without causing dislocations.

5. The TORP according to one of the preceding claims, wherein the stem (A) has a length greater than the height of the stapes (s), in such a way as to be usable for the reconstruction of the ossicular chain both when only one small bone is injured, i.e. the incus, and when two small bones are injured, i.e. the incus and the superstructure of the stapes (S).

## Patentansprüche

1. Prothese zum vollständigen Ersatz der Gehörknöchelchen (TORP) zur Rekonstruktion der Gehörknöchelchenkette, umfassend einen Kopf und einen synthetischen Schaft, wobei
- der Kopf (C) aus einem einzigen Knorpelblock gebildet ist und mit einem Loch versehen ist;
- die verbesserte TORP ferner eine Knorpelbasis (B) umfasst, die mit einem Loch versehen ist und die gleiche Dicke wie der Kopf (C), jedoch eine kleinere Fläche aufweist;
- der synthetische Schaft (A) eine zylindrische Form aufweist und beidseitig jeweils mit einer stumpfen Spitze (A1, A2) versehen ist, wobei die stumpfen Spitzen (A1, A2) in den jeweiligen Löchern des Kopfes (C) und der Basis (B) eingesetzt sind;
wobei die TORP derart ausgestaltet ist, dass der Kopf (C) unterhalb des Trommelfells (D) und die Basis (B) oberhalb der Fußplatte (E) des Steigbügels (S) positioniert werden können, sodass während des Heilungsprozesses das Perichondrium des Kopfes (C) mit dem Trommelfell (D) und das Perichondrium der Basis (B) mit der Schleimhaut der Fußplatte (E) verwächst.

2. TORP nach dem vorhergehenden Anspruch, wobei die Spitze (A1) an einem Ende des Schafts (A) derart ausgestaltet ist, dass sie vollständig in den Kopf (C) eingesetzt werden kann, der aus einem mit Perichondrium bedeckten Knorpelblock gebildet ist, während die Spitze (A2) am gegenüberliegenden Ende des Schafts (A) derart ausgestaltet ist, dass sie vollständig in die Basis (B) eingesetzt werden kann, die aus einem Knorpelblock gebildet ist, der kleiner als derjenige des Kopfes (C) ist.

3. TORP nach einem der vorhergehenden Ansprüche, wobei sie eine Länge von 5 oder 6 oder 7 mm aufweist und aus Folgendem besteht:
• einem mit Perichondrium bedeckten Knorpelkopf (C) mit einer Dicke von 1 mm und einer Fläche von 2,5 × 2,5 mm;
• einem synthetischen zylindrischen Schaft (A) aus Gold oder Platin oder Titan oder Titan und Polytetrafluorethylen mit einem Durchmesser von 0,4 mm, der an beiden Enden (A1, A2) jeweils mit einer 1 mm langen stumpfen Spitze (A1, A2) mit einem Durchmesser von 0,2 mm versehen ist, wobei der Schaft (A) eine Länge aufweist, die gleich ist:
- 3 mm bei einer 5 mm langen TORP,
- 4 mm bei einer 6 mm langen TORP,
- 5 mm bei einer 7 mm langen TORP;
• einer mit Perichondrium bedeckten Knorpelbasis (B) mit einer Dicke von 1 mm und einer Fläche von 0,8 × 0,8 mm.

4. TORP nach einem der vorhergehenden Ansprüche, wobei sie derart ausgestaltet ist, dass die Länge der stumpfen Spitzen (A1, A2) so bemessen ist, dass bei Schwankungen des atmosphärischen Drucks die an den beiden Enden vorhandenen stumpfen Titanspitzen (A1, A2) in jedem Fall zumindest teilweise in den jeweiligen Löchern des Kopfes und der Basis eingesetzt bleiben, ohne Dislokationen zu verursachen.

5. TORP nach einem der vorhergehenden Ansprüche, wobei der Schaft (A) eine Länge aufweist, die größer als die Höhe des Steigbügels (S) ist, sodass sie zur Rekonstruktion der Gehörknöchelchenkette sowohl dann verwendet werden kann, wenn nur eines der kleinen Gehörknöchelchen, nämlich der Amboss, geschädigt ist, als auch dann, wenn zwei kleine Gehörknöchelchen, nämlich der Amboss und der Steigbügeloberbau (S), geschädigt sind.

## Revendications

1. Prothèse de remplacement ossiculaire total (TORP) pour la reconstruction de la chaîne ossiculaire, comprenant une tête et une tige synthétique, dans laquelle
- ladite tête (C) est formée d'un seul bloc cartilagineux et est pourvue d'un trou;
- ladite TORP améliorée comprend en outre une base cartilagineuse (B), pourvue d'un trou, ayant la même épaisseur que la tête (C), mais avec une surface plus petite;
- ladite tige synthétique (A) présente une forme cylindrique et est pourvue, de part et d'autre, d'une pointe mousse respective (A1, A2), dans laquelle lesdites pointes mousses (A1, A2) sont insérées dans les trous respectifs de la tête (C) et de la base (B);
ladite TORP est configurée de manière à pouvoir positionner, respectivement, ladite tête (C) sous la membrane tympanique (D) et ladite base (B) au-dessus de la platine (E) de l'étrier (S); permettant ainsi d'obtenir que, pendant le processus de cicatrisation, le périchondre de la tête (C) soit lié à la membrane tympanique (D) et le périchondre de la base (B) soit lié à la muqueuse de la platine (E).

2. TORP selon la revendication précédente, dans laquelle la pointe (A1) d'une extrémité de la tige (A) est configurée pour être entièrement insérée dans ladite tête (C), qui est formée d'un bloc cartilagineux recouvert d'un périchondre, tandis que la pointe (A2) de l'extrémité opposée de la tige (A) est configurée pour être entièrement insérée dans ladite base (B), qui est formée d'un bloc cartilagineux plus petit que celui de la tête (C).

3. TORP selon l'une des revendications précédentes, dans laquelle la longueur est de 5 ou 6 ou 7 mm et consiste en:
• une tête cartilagineuse (C), recouverte d'un périchondre, présentant une épaisseur de 1 mm et une surface de 2,5 × 2,5 mm;
• une tige cylindrique synthétique (A) en or, en platine, en titane, ou en titane et polytétrafluoroéthylène, d'un diamètre de 0,4 mm et ayant, à chacune de ses deux extrémités (A1, A2), une pointe mousse (A1, A2) d'une longueur de 1 mm et d'un diamètre de 0,2 mm, ladite tige (A) présentant une longueur égale à:
- 3 mm pour la TORP d'une longueur de 5 mm,
- 4 mm pour la TORP d'une longueur de 6 mm,
- 5 mm pour la TORP d'une longueur de 7 mm;
• une base cartilagineuse (B), recouverte d'un périchondre, présentant une épaisseur de 1 mm et une surface de 0,8 × 0,8 mm.

4. TORP selon l'une des revendications précédentes, dans laquelle elle est configurée de manière que la longueur des pointes mousses (A1, A2) soit telle que, en cas de variations de la pression atmosphérique, lesdites pointes mousses en titane (A1, A2), présentes aux deux extrémités, restent dans tous les cas au moins partiellement insérées dans les trous respectifs de la tête et de la base, sans provoquer de dislocations.

5. TORP selon l'une des revendications précédentes, dans laquelle la tige (A) présente une longueur supérieure à la hauteur de l'étrier (S), de manière à pouvoir être utilisée pour la reconstruction de la chaîne ossiculaire aussi bien lorsqu'un seul osselet est lésé, à savoir l'enclume, que lorsque deux osselets sont lésés, à savoir l'enclume et la superstructure de l'étrier (S).
